# EUROPÄISCHE PATENTSCHRIFT

(11) **EP 0 948 321 B1**
(45) Veröffentlichungstag und Bekanntmachung des Hinweises auf die Patenterteilung: **22.10.2003**
(21) Anmeldenummer: 97953773.5
(22) Anmeldetag: 10.12.1997
(51) Int. Cl.: A61K 9/26, A61K 9/16

(54) **ZUBEREITUNG IN FORM EINES WAHLWEISE WIRKSTOFFHALTIGEN MATRIXMATERIAL-HILFSSTOFF COMPOUNDS**
PREPARATION IN FORM OF A MATRIX MATERIAL-AUXILIARY AGENT COMPOUND CONTAINING OPTIONALLY AN ACTIVE SUBSTANCE
PREPARATION SOUS FORME D'UN COMPOSE MATERIAU MATRICIEL-MATIERE AUXILIAIRE RENFERMANT EVENTUELLEMENT UNE MATIERE ACTIVE

(30) Priorität: 12.12.1996 DE 19651734; 12.11.1997 DE 19749897
(43) Veröffentlichungstag der Anmeldung: 13.10.1999
(73) Patentinhaber: DDS DRUG DELIVERY SERVICE GESELLSCHAFT ZUR FÖRDERUNG DER FORSCHUNG IN PHARMAZEUTISCHER TECHNOLOGIE UND BIOPHARMAZIE MBH, 24119 Kronshagen (DE)
(72) Erfinder: MÜLLER, Rainer, H., D-12161 Berlin (DE)
(74) Vertreter: UEXKÜLL & STOLBERG
(86) Internationale Anmeldenummer: EP9706893
(87) Internationale Veröffentlichungsnummer: WO98025590

(56) Entgegenhaltungen:
- EP-A- 0 192 173
- EP-A- 0 682 945
- WO-A-94/00111
- DE-C- 3 506 276
- DE-C- 4 122 591
- DATABASE WPI Week 9722 Derwent Publications Ltd., London, GB; AN 97-241649 XP002073923 & JP 09 077 669 A (TAKEDA CHEM IND LTD) , 25. März 1997

## Beschreibung

Die Erfindung bezieht sich auf eine Zubereitung in Form eines Compounds gemäß Anspruch 1 oder 2, die eine Hilfsstoffphase mit wenigstens einem Hilfsstoff und/oder eine Wirkstoffphase mit wenigstens einem Wirkstoff und eine Phase eines matrixbildenden Materials (im folgenden auch Matrixmaterial genannt) ausgewählt aus Polymer und/oder Lipid, d.h. eine polymere Phase und/oder eine Lipidphase mit wenigstens einem Polymer bzw. Lipid aufweist, und damit auf polymer- und/oder lipidhaltige Retardarzneiformen, Verfahren zu deren Herstellung und deren Verwendung, insbesondere zur Herstellung von Tabletten oder anderen größeren Matrixeinheiten.

Derartige Compounds sind physikalische Verbindungen von mindestens zwei Ausgangsstoffen und werden insbesondere im pharmazeutischen Bereich eingesetzt.

Es ist bekannt, zur Erreichung einer kontrollierten, verzögerten oder von physiologischen Parametern unabhängigen Freisetzung von

Wirkstoffen aus einer Zubereitung, die Ausgangsstoffe derart zu verarbeiten, daß die resultierenden Zubereitungen bzw. die aus diesen Zubereitungen hergestellten Arnzeiformen einen die Freisetzung steuernden Überzug aufweisen (z.B. aus Polymeren wie Polymethacrylate oder organischen Molekülen wie Schellack oder Celluloseacetatphthalat) bzw. alternativ ein aus Polymeren bestehendes Matrixsystem aufweisen.

Unter Verwendung von Polymeren hergestellte, in der Literatur beschriebene Matrixeinheiten zur kontrollierten Freisetzung sind:
1. Polymerpartikel
   (z.B. Pellets, Granulatkörner, Mikropartikel)
2. größere Matrixeinheiten
   (z.B. Tabletten, Drageekerne und Implantate).

Die im folgenden näher beschriebenen Partikel sind dadurch gekennzeichnet, daß der Wirkstoff molekulardispers oder partikulär in der polymeren Phase eingebettet ist.

Die im folgenden näher beschriebenen größeren Matrixeinheiten müssen in der Regel durch das kostenaufwendige Verfahren der Komprimierung nach vorheriger Granulation hergestellt werden.

### Arzneiformen zur kontrollierten Freisetzung unter Verwendung von Polymeren:

Der die Freisetzung kontrollierende Effekt einer solchen Zubereitung oder Arnzeiform, auch "Controlled Release"-Zubereitung (CR-Zubereitung) genannt, wird einerseits durch die Eigenschaften der polymeren Phase selbst, wie beispielsweise die Benetzbarkeit, die Quellbarkeit oder die Kristallinität, und andererseits durch die Struktur der durch die polymere Phase gebildeten Matrix gesteuert. Diese Matrixstruktur, die homogen oder heterogen ausgebildet sein kann, ist entweder bereits in der Zubereitung selbst vorhanden oder entsteht während der Verarbeitung bei der Zubereitung zur Arzneiform.

Als die Freisetzung beeinflussende Eigenschaften der polymeren Phase seien hier die Löslichkeitseigenschaften genannt. So sind Polymere bzw. Makromoleküle aufgrund ihrer Unlöslichkeit und/oder Quellbarkeit in wäßrigen Lösungsmitteln geeignet, Wirkstoffe, die in einer Matrix solcher Polymere bzw. Makromoleküle eingebettet sind, durch die Poren der Matrix verzögert freizusetzen. Weiterhin sind Arzneiformen mit Polymerstoffen bekannt, die aufgrund der Löslichkeit der Polymere im Magen- oder Darmsaft eine den Ort der Freisetzung kontrollierende Zubereitung darstellen.

Bei diesen die Wirkstofffreisetzung kontrollierenden Zubereitungen sind insbesondere zwei Gruppen zu unterscheiden.

Einerseits sind polymerhaltige Partikel in einer Größenordnung von ca. 0,01 bis 2 mm bekannt, die auch als Mikropartikel (0,05 bis 0,2 mm), Granulatkörner oder Pellets bezeichnet werden. Aber auch die erst seit kürzerer Zeit bekannten Mikropartikel bzw. Mikrosphärulen mit einer typischen Größe von 50 bis 200 µm, Nanopartikel, Nanopellets und Nanosphärulen werden, sofern sie eine polymere Phase aufweisen, der Gruppe der polymerhaltigen Partikel zugeordnet. Die Partikel liegen als eigenständige Freisetzungseinheit in Form einer partikulären Matrix vor, wobei dann bereits die Zubereitung eine Matrixstruktur aufweist.

Andererseits können die in der vorliegenden Anmeldung beschriebenen Partikel zu größeren Freisetzungseinheiten bzw. größeren Matrixeinheiten vereinigt werden. Diese Weiterverarbeitung wird weiter unten im einzelnen ausgeführt.

Als Beispiele der partikulären Matrices, deren Partikel eigenständige Freisetzungseinheiten bilden, seien die Dispersion von Mikropartikeln zur parenteralen Injektion, die eine kontrollierte Freisetzung von LH-RH-Analoga erlauben, sowie die Füllung von Pellets in eine Gelatinekapsel bei Handelspräparaten wie Sympathomimetika genannt. Diese werden von Müller, R.H., Hildebrand G.E. (Hrsg.) in "Pharmazeutische Technologie: Moderne Arzneiformen", Wissenschaftliche Verlagsgesellschaft mbh Stuttgart, (1997), von Bauer, K.H., Frömming, K-H., Führer, C. in "Pharmazeutische Technologie"; Georg Thieme Verlag Stuttgart, New York, (1991), sowie von List, P.H. "Arzneiformenlehre" Wissenschaftliche Verlagsgesellschaft mbh Stuttgart, (1986), beschrieben.

Weiterhin sind in der EP 0 261 677 polymerhaltige Zusammensetzungen beschrieben, die eine verzögerte Freisetzung des Wirkstoffs ermöglichen sollen. Zur Herstellung dieser Zusammensetzungen wird ein Sprühtrocknungsverfahren offenbart, so daß unter Anwendung der Lehre dieser Druckschrift Partikel mit einer Größe von mindestens 30 µm erhalten werden, die den Wirkstoff in gleichmäßiger Verteilung aufweisen.

Zur Herstellung solcher Zubereitungen mit partikulärer Matrixstruktur werden in der Literatur mehrere Verfahren beschrieben.

Bei den Verfahren nach der "solvent evaporation"- oder der "inliquid-drying"-Methode ist das Polymer bzw. der Matrixbildner eine in einem organischen Lösungsmittel lösliche Substanz (z.B. Polymere wie Polylactide, Polylactid/Glycolid). Das Polymer wird in einem organischen Lösungsmittel gelöst, der Wirkstoff wird ebenfalls in der organischen Phase gelöst oder - im Falle unlöslicher Wirkstoffe - dispergiert. Die den Wirkstoff enthaltende Lösung des Polymers bzw. Matrixbildners wird dann in eine wäßrige Tensidlösung gegeben und durch Rühren eine O/W-Emulsion hergestellt. Das organische Lösungsmittel wird dann entfernt und der Matrixbildner präzipitiert. Es entstehen feste Pellets bzw. Mikropartikel. Je nach der Methode zur Entfernung des Lösungsmittels unterscheidet man zwischen der "solvent evaporation"und der "in-liquid-drying"-Methode.

Diese Verfahren wurden von Speiser, P. in Müller, R.H., Hildebrand, G.E. (Hrsg.) "Pharmazeutische Technologie: Moderne Arzneiformen", Wissenschaftliche Verlagsgesellschaft mbh Stuttgart (1997), von Beck, L.R., Pope, V.Z., Cowsar, D.R., Lewis, D.H., Tice, T.R. in "Evulation of a new three-month injectable contraceptive microsphere system in primates (baboons)", Contracept. Deliv.1 Syst., 1, 79-80 (1980), von Beck, L.R., Flowers, C.E., Pope, V.Z., Tice, T.R. Wilborn, W.H. in "Clinical evaluation of an improved injectable microcapsule contraceptive system" in Amer. J. Obstet. Gynecol. 147 (7), 815-821 (1983) und von Beck, L.R., Pope, V.Z., Flowers, C.E., Cowsar, D.R., Tice, T.R., Lewis, D.H., Dunn, R.L., Moore, A.B., Gilley, R.M. in "Poly(d,l-lactide-coglycolide)/norethisterone microcapsules: An injectable biogradable contraceptive" in Biol. Reprod. 28, 186-195 (1983a) beschrieben.

Mit diesen Verfahren können sehr feine Partikel im Bereich von wenigen Mikrometern erhalten werden. Nachteilig ist jedoch der große Aufwand, mit dem die Herstellungsmethode verbunden ist, sowie die Belastung der Partikel mit Restlösungsmittel. Aus diesem Grund gibt es auch bisher in Deutschland noch kein Produkt, das nach einer dieser Verfahren hergestellt worden ist und die Zulassungskriterien für ein Arzneimittel erfüllt.

Alternativ kann man die den Wirkstoff enthaltende Lösung des Polymers bzw. des Matrixbildners-versprühen. Auch hier ist ein Restgehalt an organischen Lösungsmitteln im Produkt aufgrund des Herstellungsverfahrens nicht zu vermeiden. Nach diesem Verfahren hergestellte Produkte, wie z.B. Mikropartikel zur parenteralen Applikation von Bromocriptin, werden von Fahr, A., Kissel, T. in Müller, R.H., Hildebrand, G.E. (Hrsg.), "Pharmazeutische Technologie: Moderne Arzneiformen", Wissenschaftliche Verlagsgesellschaft mbh Stuttgart, (1997) beschrieben. Sie sind auf dem pharmazeutischen Markt. Das Problem des Restlösungsmittelgehalts ist jedoch nur dadurch verdrängt worden, daß hier auch die Freisetzung des toxischen Lösungsmittels verzögert und damit in geringer Menge erfolgt. Mit der pro Tag aus der Matrix freigegebenen Menge bleibt man unter dem maximalen täglich tolerierten Wert.

Alle bisher genannten Verfahren sind dadurch gekennzeichnet, daß die polymere Phase bzw. der Matrixbildner in einer gelösten Form als Molekül vorliegt und sich in einem organischen Lösungsmittel befindet. Es entstehen partikuläre Zubereitungen, deren polymere Phase den Wirkstoff molekulardispers oder in Form feiner Partikel enthält. Diese Zubereitungen weisen eine sogenannte heterogene Matrixstruktur auf, wie auch von Fahr, A., Kissel, T. in Müller, R.H., Hildebrand, G.E. (Hrsg.), "Pharmazeutische Technologie: Moderene Arnzeiformen", Wissenschaftliche Verlagsgesellschaft mbh Stuttgart, (1997) beschrieben ist.

Ein weiteres Verfahren zur Herstellung einer partikulären Zubereitung mit polymerer Phase unter Vermeidung der Verwendung von organischen Lösungsmittel wird in der EP 0 361 677 dargestellt. Der nach dieser Druckschrift wasserlösliche Matrixbildner bzw. die polymere Phase wird in Wasser gelöst (z.B. Ethylacrylat/Methacrylat-Copolymer in ammoniakalischer Lösung), der Wirkstoff wird ebenfalls gelöst oder dispergiert und - im Gegensatz zur "solvent evaporation"- und "in liquid-drying"-Methode - anstatt einer O/W - nun eine W/O-Emulsion hergestellt. Dispersionsmedien sind mit Wasser nicht mischbare organische Lösungsmittel, z.B. flüssiges Paraffin oder Methylenchlorid. Der Matrixbildner kann in Wasser gelöst oder auch in der Wasserphase emulgiert werden. Im zweiten Fall wird eine Emulsion in einem mit Wasser nicht mischbaren organischen Lösungsmittel dispergiert. Durch aufwendige azeotrope Destillation von Wasser und organischem Lösungsmittel werden Polymerpartikel ausgefällt, die den Wirkstoff in molekulardisperser oder partikulärer Verteilung einschließen. Die Gewinnung der Partikel erfolgt durch Separation mittels Filtration und anschließendes Waschen.

In der US-A-5 043 280 wird ein Verfahren zur Herstellung einer partikulären Zubereitung durch Extraktion in überkritischen Gasen beschrieben. Hierbei ist der Matrixbildner - wie bei der "solvent evaporation" - eine in einem organischen Lösungsmittel lösliche Substanz, wie z.B. ein Polymer. Das Polymer wird in einem organischen Lösungsmittel gelöst, und der Wirkstoff wird ebenfalls gelöst oder - im Falle unlöslicher Wirkstoffe - in der organischen Phase dispergiert. Die den Wirkstoff enthaltende Lösung des Matrixbildners wird dann in einer überkritischen Gasphase fein versprüht. Feine Tropfen verteilen sich im überkritischen Gas, das das organische Lösungsmittel aus den Tropfen extrahiert. Als Folge kommt es zur Präzipitation von Partikeln, die den Wirkstoff enthalten.

Auch diese genannten Verfahren führen zu Zubereitungen, die den Wirkstoff in molekulardisperser bzw. partikulärer Form in der polymeren Phase eingebettet aufweisen. Durch diesen verfahrensbedingten Einschluß des Wirkstoffs in die polymere Phase weist die Außenphase der Zubereitung größtenteils Polymer auf, wodurch auch die pharmazeutischen Eigenschaften, die für eine eventuelle Weiterverarbeitung von Bedeutung sind, festgelegt werden. Ferner weisen die genannten Zubereitungen den Nachteil auf, daß sie nur unter erheblichem Kosten- und Zeitaufwand herstellbar sind.

DE-A-35 06 276 offenbart ein Verfahren zur Herstellung von Direkttablettiermitteln, wobei Cellulosepulver mit einer heißen Lösung von 50%-iger Lactose in Wasser vermischt und die so erhaltene Mischung absekühlt wird. Die so erhaltene Masse wird anschließend grannliert und getrocknet. Alternativ kann eine Mischung von mikrokristalliner Lactose mit Cellulose pulver in kattern Wasser aufgeschlämmt und anschlieißend sprühgetrocknet werden.

Die Möglichkeit der Weiterverarbeitung partikulärer, polymerhaltiger Zubereitungen zu Arzneiformen, die größere Matrixeinheiten aufweisen, wie beispielsweise zu Tabletten, Drageekernen oder Implantaten ist bekannt. So wird von Müller, R.H., Hildebrand, G.E. (Hrsg.) in Pharmazeutische Technologie: Moderne Arzneiformen", Wissenschaftliche Verlagsgesellschaft mbh Stuttgart, (1997), die Herstellung von LH-RH-Analoga enthaltenden Implantaten beschrieben. Von besonderer Bedeutung ist dabei die Herstellung von Tabletten, da diese Arzneiform viele Vorteile aufweist, wie beispielsweise die Möglichkeit zur Verarbeitung fast aller festen Wirkstoffe, die hohe Dosierungsgenauigkeit, die einfache Einnahme und Handhabung und die gute Lager- und Transportfähigkeit.

Die Herstellung von Arzneiformen, die größere Matrixeinheiten darstellen, und insbesondere von Tabletten, erfolgt üblicherweise durch Komprimierung. Dabei sind zur Verarbeitung der herkömmlichen polymerhaltigen Zubereitungen in Form der partikulären Matrices mehrere Verfahrensschritte notwendig.

Zuerst werden die verschiedenen Inhaltsstoffe, wie beispielsweise verschiedene Wirkstoffe, Hilfsstoffe und Polymere homogen vermischt. Anschließend wird die Mischung einer Feuchtgranulation durch Zusatz von Binde-, Kleb- oder Lösungsmitteln unterzogen. Das resultierende Granulat wird zum Entzug der Restfeuchte getrocknet. Die Komprimierung zu Tabletten, Drageekernen oder Implantaten erfolgt dann mit dem trockenen Granulat unter Zusatz von weiteren Hilfsstoffen, wie Fließregulierungs-, Schmier- und Formtrennmitteln.

Nachteilig ist, daß der Wirkstoff während der Feuchtgranulation über lange Zeit der Feuchtigkeit des Binde-, Kleb- oder Lösungsmittels ausgesetzt wird und während des notwendigen Trocknungsverfahrens zwingend einer erhöhten Temperatur ausgesetzt wird. Weiterhin ist das Verfahren aufgrund der verschiedenen Einzelschritte und hierbei benötigter Vorrichtungen und Geräte mit relativ großem Zeitaufwand verbunden und somit kostenintensiv.

Die Direkttablettierung von Zubereitungen mit polymeren Bestandteilen, die zur Herstellung von Tabletten ohne polymere Phase aufgrund der niedrigen Kosten und der schnellen Durchführbarkeit bereits häufig angewendet wird, ist bisher aufgrund folgender Schwierigkeiten nicht möglich gewesen.

Zum einen weisen die Polymere durch überwiegend elastische Verformung ein schlechtes Komprimierverhalten auf, da eine Komprimierung üblicherweise hauptsächlich durch plastische Verformung erreicht wird.

Zum anderen neigt die Tablettiermischung zu einer unerwünschten Entmischung zwischen pulverisierten Wirkstoffen und/oder Hilfsstoffen und Polymeren aufgrund der unterschiedlichen Oberflächenbeschaffenheit und der daraus resultierenden unterschiedlichen Fließeigenschaften. Bei der Direkttablettierung würden daher durch die fortschreitende Entmischung des Tablettierguts stark inhomogene Tabletten erhalten werden.

Ein weiteres Problem ist das allgemein schlechte Fließverhalten der Polymere. Dies hat zur Folge, daß ein zufriedenstellender Retardeffekt durch die begrenzte Beimischungsfähigkeit von Polymeren zur Tablettiermischung nicht erreicht wird. In der Literatur wird von McGinity, J.W., Cameron, C.G., Cuff. G.W. in "Controlled-release teophylline tablet formulations containing acrylic resins. I. Dissolution properties of tablets", Drug Development and Industrial Pharmacy, 9(162), 57-68 (1983) und von Cameron, C.G., McGinity, J.W. in "Controlled-release teophylline tablet formulations containing acrylic resins. II. Combination resin formulations" und "III. Influence of filler excipient"., a.a.O. 13(8), 1409-1427 (1987), a.a.O. 13(2), 303-318 (1987) bei Acrylatpolymeren ein in der Regel maximaler Zusatz von 10 - 15% Polymer in einer Tablettenrezeptur zur Direkttablettierung beschrieben.

Es sind aber auch Retardarzneiformen bekannt, in denen Lipide verwendet werden. Bei solchen in der Literatur beschriebenen Arzneiformen zur kontrollierten Freisetzung unter Verwendung von Lipiden handelt es sich im wesentlichen um:
1. Suppositorien
2. Vaginalglobuli
3. Pellets zur peroralen Applikation (z.B. Mucosolvan retard).

Im Vergleich zu Polymeren bieten Lipide folgende Vorteile:
1. gute Verträglichkeit in vivo, insbesondere wenn sie aus physiologischen Fettsäuren aufgebaut sind
2. keine toxikologisch bedenklichen Rückstände aus der Produktion (z.B. Katalysatorrückstände)
3. Steuerung der Abbaugeschwindigkeit über chemische Struktur der Lipide
4. kostengünstig

Somit sind sie neben Polymeren zur Herstellung von CR-Formulierungen einsetzbare Hilfsstoffe.

### Arzneiformen zur kontrollierten Freisetzung unter Verwendung von Lipiden - Zubereitungen aus Compounds:

Die Herstellung von Suppositorien und Vaginalglobuli erfolgt in der Regel durch Ausgießen der arzneistoffhaltigen Mischung (P.H. List, Arzneiformenlehre, Wissenschaftliche Verlagsgesellschaft 1976).

Die Herstellung von Suppositorien ist auch durch Komprimieren einer Mischung von Lipidpartikeln und Arzneistoffpulver möglich, eine großtechnische Herstellung bereitet jedoch aufgrund der in der Regel schlechten Fließfähigkeit dieser Mischungen beim Abfüllen in die Preßformen Schwierigkeiten. Primär wird diese Methode daher für die Kleinherstellung im Rezepturmaßstab in der Apotheke beschrieben (K. Münzel, J. Büchi, O.-E. Schultz, Galenisches Praktikum, Wissenschaftliche Verlagsgesellschaft Stuttgart, S. 652, 1959). Eingesetzt werden dabei nur Lipide die bei Körpertemperatur schmelzen oder zumindest erweichen.

Arzneiformen zur peroralen Applikation sind Pellets, die großtechnisch durch Extrusion geschmolzener Lipide mit einem Extrudor und einer Lochscheibe hergestellt werden (Voigt, Lehrbuch der Pharmazeutischen Technologie, Verlag Chemie, 1975). Nachteilig sind hierbei z.B. die Einarbeitung der Arzneistoffe in das Lipid (z.B. durch Dispergieren oder Lösen), die Thermobelastung der Arzneistoffe bei der Extrusion und die Notwendigkeit der Weiterverarbeitung der Pellets in einem zusätzlich Produktionsschritt (z.B. Einfüllung in Hartgelatinekapseln).

Die Aufgabe der vorliegenden Erfindung liegt nun darin, eine Zubereitung in Form eines matrixmaterialhaltigen Compounds als Retardarzneizubereitungen zu Verfügung zu stellen, die eine Hilfsstoff- und/oder eine Wirkstoffphase und eine Matrixmaterialphase aufweist. Die Zubereitung soll einen ausreichend großen Matrixmaterialanteil aufweisen, so daß eine kontrollierte Freisetzung des enthaltenen oder bei einer Verarbeitung zu größeren Matrixeinheiten nachträglich hinzugefügten Wirkstoffs ermöglicht wird. Außerdem soll die Zubereitung mittels Direkttablettierung zu größeren Matrixeinheiten verarbeitet werden können. Ferner soll ein Verfahren zur Herstellung dieser Zubereitung bzw. Compounds angegeben werden.

Die erfindungsgemäße Aufgabe wird durch eine matrixmaterialhaltige Retardarzneiform gelöst, die in Form eines Matrixmaterial-Hilfsstoff-Compounds, Matrixmaterial-Wirkstoff-Compounds und/oder Matrixmaterial-Hilfsstoff-Wirkstoff-Compounds vorliegen, wobei das Matrixmaterial ausgewählt ist aus Polymeren und Lipiden, so daß der Compound eine polymere Phase und/oder Lipidphase und eine Hilfsstoff- und/oder eine Wirkstoffphase aufweist.

Ein solcher Compound kann durch Direktkomprimierung in seine endgültige Arzneimittelform überführt werden.

Die Erfindung bezieht sich somit auf polymer- oder lipidhaltige Zubereitungen, die
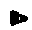 in Form eines Compounds vorliegen, der eine polymere bzw. lipide Phase mit wenigstens einem Polymer bzw. Lipid, eine Hilfsstoffphase mit wenigstens einem Hilfsstoff und/oder eine Wirkstoffphase mit wenigstens einem Wirkstoff aufweist.

Erfindungsgemäß ist erkannt worden, daß die Lösung der Aufgabe durch die in Anspruch 1 beschriebene Zubereitung möglich ist, die
a) eine Hilfsstoffphase mit wenigstens einem Hilfsstoff und/oder eine Wirkstoffphase mit wenigstens einem Wirkstoff und eine polymere Phase mit wenigstens einem Polymer aufweist, wobei die polymere Phase inkohärent ist und die Hilfs- und/oder Wirkstoffphase kohärent ist, oder
b) eine Lipidphase mit wenigstens einem Lipid, eine Hilfsstoffphase mit wenigstens einem Hilfsstoff und/oder eine Wirkstoffphase mit wenigstens einem Wirkstoff aufweist, wobei die Lipid-phase inkohärent ist und die Hilfs- und/oder Wirkstoffphase kohärent ist.

Insbesondere kann die Polymerphase bzw. die Lipidphase Hilfsund/oder Wirkstoff enthält oder frei davon sein.

Bei der erfindungsgemäßen Zubereitung kann der Anteil an polymerphase bzw. Lipidphase bezogen auf die Gesamtmenge von Hilfsstoff- und/oder Wirkstoffphase und Polymerphase bzw. Lipidphase zwischen 1 und 98% betragen.

Insbesondere kann die Zubereitung einen Anteil an Polymer-/Lipidphase von 10 bis 95% aufweisen.

Ferner kann der Anteil der Polymer-/Lipidphase in der Zubereitung mehr als 15% und höchstens 90% betragen.

Besonders vorteilhaft zur Ausführung der vorliegenden Erfindung ist es aber, wenn die Polymer-/Lipidphase einen Anteil von 40 bis 70% bezogen auf die Gesamtmenge von Hilfsstoff- und/oder Wirkstoffphase und Polymer-/Lipidphase aufweist.

Die erfindungsgemäße Zubereitung kann grundsätzlich jede Art von Wirkstoff aufweisen oder frei von Wirkstoff sein. Ferner kann der Wirkstoff der Zubereitung nachträglich, z.B. vor einer Weiterverarbeitung zu größeren Matrixeinheiten zugesetzt werden. Im allgemeinen kann die Zubereitung folgende Wirkstoffgruppen enthalten:
- hydroxylierte Kohlenwasserstoffe
- Carbonylverbindungen wie Ketone (z.B. Haloperidol), Monosaccharide, Disaccharide und Aminozucker
- Carbonsäuren wie aliphatische Carbonsäuren, Ester aliphatischer und aromatischer Carbonsäuren, basisch substituierte Ester aliphatischer und aromatischer Carbonsäuren (z.B. Atropin, Scopolamin), Lactone (z.B. Erythromycin), Amide und Imide aliphatischer Carbonsäuren, Aminosäuren, aliphatische Aminocarbonsäuren, Peptide (z.B. Ciclosporin), Polypeptide, β-Lactamderivate, Penicilline, Cephalosporine, aromatische Carbonsäuren (z.B. Acetylsalicylsäure), Amide aromatischer Carbonsäuren, vinyloge Carbonsäuren und vinyloge Carbonsäureester
- Kohlensäurederivate wie Urethane und Thiourethane, Harnstoff und Harnstoffderivate, Guanidinderivate, Hydantoine, Barbitursäurederivate und Thiobarbitursäurederivate
- Nitroverbindungen wie aromatische Nitroverbindungen und heteroaromatische Nitroverbindungen
- Amine wie aliphatische Amine, Aminoglykoside, Phenylalkylamine, Ephedrinderivate, Hydroxyphenylethanolamine, Adrenalinderivate, Amfetaminderivate, aromatische Amine und Derivate, quartäre Ammoniumverbindungen
- schwefelhaltige Verbindungen wie Thiole und Disulfane, Sulfone, Sulfonsäureester und Sulfonsäureamide
- Polycarbocyclen wie Tetracycline, Steroide mit aromatischem Ring A, Steroide mit alpha,beta-ungesättigter Carbonylfunktion im Ring A und alpha-Ketol-Gruppe (oder Methylketo-Gruppe) am C-17, Steroide mit einem Butenolid-Ring am C-17, Steroide mit einem Pentadienolid-Ring am C-17 und Seco-Steroide
- O-haltige Heterocyclen wie Chromanderivate (z.B. Cromoglicinsäure)
- N-haltige Heterocyclen wie Pyrazolderivate (z.B. Propyphenazon, Phenylbutazon)
- Imidazolderivate (z.B. Histamin, Pilocarpin), Pyridinderivate (z.B. Pyridoxin, Nicotinsäure), Pyrimidinderivate (z.B. Trimetoprim), Indolderivate (z.B. Indometacin), Lysergsäurederivate (z.B. Ergotamin), Yohimbanderivate, Pyrrolidinderivate, Purinderivate (z.B. Allopurinol), Xanthinderivate, 8-Hydroxychinolinderivate, Amino-hydroxy-alkylierte Chinoline, Aminochinoline, Isochinolinderivate (z.B. Morphin, Codein), Chinazolinderivate, Benzopyridazinderivate, Pteridinderivate (z.B. Methotrexat), 1,4-Benzodiazepinderivate, tricyclische N-haltige Heterocyclen, Acridinderivate (z.B. Ethacridin) und Dibenzazepinderivate (z.B. Trimipramin)
- S-haltige Heterocyclen wie Thioxanthenderivate (z.B. Chlorprothixen)
- N,O- und N,S-haltige Heterocyclen wie monocyclische N,0-haltige Heterocyclen, monocyclische N,S-haltige Heterocyclen, Thiadiazinderivate, bicyclische N-S-haltige Heterocyclen, Benzothiadiazinderivate, tricyclische N,S-haltige Heterocyclen und Phenothiazinderivate
- O,P,N-haltige Heterocyclen (z.B. Cyclophosphamid)

Die folgenden Arzneistoffe (als Salz, Ester, Ether oder in freier Form) sind beispielsweise für eine Einarbeitung geeignet:
Analgetika/Antirheumatika
   BTM Basen wie Morphin, Codein, Piritamid, Fentanyl und Fentanylderivate, Levomethadon, Tramadol, Diclofenac, Ibuprofen, Indometacin, Naproxen, Piroxicam, Penicillamin
Antiallergika
   Pheniramin, Dimetinden, Terfenadin, Astemizol, Loratidin, Doxylamin, Meclozin, Bamipin, Clemastin
Antibiotika/Chemotherapeutika
   hiervon: Polypeptidantibiotika wie Colistin, Polymyxin B, Teicplanin, Vancomycin; Malariamittel wie Chinin, Halofantrin, Mefloquin, Chloroquin, Virustatika wie Ganciclovir, Foscarnet, Zidovudin, Aciclovir und andere wie Dapson, Fosfomycin, Fusafungin, Trimetoprim
Antiepileptika
   Phenytoin, Mesuximid, Ethosuximid, Primidon, Phenobarbital, Valproinsäure, Carbamazepin, Clonazepam
Antimykotika
   a) intern:
      Nystatin, Natamycin, Amphotericin B, Flucytosin, Miconazol, Fluconazol, Itraconazol
   b) extern außerdem:
      Clotrimazol, Econazol, Tioconazol, Fenticonazol, Bifonazol, Oxiconazol, Ketoconazol, Isoconazol, Tolnaftat
Corticoide (Interna)
   Aldosteron, Fludrocortison, Betametason, Dexametason, Triamcinolon, Fluocortolon, Hydroxycortison, Prednisolon, Prednyliden, Cloprednol, Methylpredinsolon
Dermatika
   a) Antibiotika:
      Tetracyclin, Erythromycin, Neomycin, Gentamycin, Clindamycin, Framycetin, Tyrothricin, Chlortetracyclin, Mipirocin, Fusidinsäure
   b) Virustatika wie oben, außerdem:
      Podophyllotoxin, Vidarabin, Tromantadin
   c) Corficoide wie oben, außerdem:
      Amcinonid, Flupredniden, Alclometason, Clobetasol, Diflorason, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid
Diagnostika
   a) radioaktive Isotope wie Te99m, In111 oder I131, kovalent gebunden an Lipide oder Lipoide oder andere Moleküle oder in Komplexen
   b) hochsubstituierte iodhaltige Verbindungen wie z.B. Lipide
Hämostyptika/Antihämorrhagika
   Blutgerinnungsfaktoren VIII, IX
Hypnotika, Sedativa
   Cyclobarbital, Pentobarbital, Phenobarbital, Methaqualon (BTM), Benzodiazepine (Flurazepam, Midazolam, Nitrazepam, Lormetazepam, Flunitrazepam, Triazolam, Brotizolam, Temazepam, Loprazolam)
Hypophysen-, Hypothalamushormone, regulatorische Peptide und ihre Hemmstoffe
   Corticotrophin, Tetracosactid, Choriongonadotropin, Urofollitropin, Urogonadotropin, Somatropin, Metergolin, Bromocriptin, Terlipressin, Desmopressin, Oxytocin; Argipressin, Ornipressin, Leuprorelin, Triptorelin, Gonadorelin, Buserelin, Nafarelin, Goselerin, Somatostatin
Immuntherapeutika und Zytokine
   Dimepranol-4-acetatamidobenzoat, Thymopentin, α-Interferon, β-Interferon, γ-Interferon, Filgrastim, Interleukine, Azathioprin, Ciclosporin
Lokalanaesthetika
   intern:
      Butanilicain, Mepivacain, Bupivacain, Etidocain, Lidocain, Articain, Prilocain,
   extern außerdem:
      Propipocain, Oxybuprocain, Tetracain, Benzocain
Migränemittel
   Proxibarbal, Lisurid, Methysergid, Dihydroergotamin, Clonidin, Ergotamin, Pizotifen
Narkosemittel
   Methohexital, Propofol, Etomidat, Ketamin, Alfentanil, Thiopental, Droperidol, Fentanyl
Nebenschilddrüsenhormone, Calciumstoffwechselregulatoren Dihydrotachysterol, Calcitonin, Clodronsäure, Etidronsäure
Opthalmika
   Atropin, Cyclodrin, Cyclopentolat, Homatropin, Tropicamid, Scopolamin, Pholedrin, Edoxudin, Idouridin, Tromantadin, Aciclovir, Acetazolamid, Diclofenamid, Carteolol, Timolol, Metipranolol, Betaxolol, Pindolol, Befunolol, Bupranolol, Levobununol, Carbachol, Pilocarpin, Clonidin, Neostimgin
Psychopharmaka
   Benzodiazepine (Lorazepam, Diazepam), Clomethiazol
Schilddrüsentherapeutika
   1-Thyroxin, Carbimazol, Thiamazol, Propylthiouracil
Sera, Immunglobuline, Impfstoffe
   a) Immunglobuline allgemein und spezifisch wie Hepatitis-Typen, Röteln, Cytomegalie, Tollwut, FSME, Varicella-Zoster, Tetanus, Rhesusfaktoren
   b) Immunsera wie Botulismus-Antitoxin, Diphterie, Gasbrand, Schlangengift, Skorpiongift
   c) Impfstoffe wie Influenza, Tuberkulose, Cholera, Diphterie, Hepatitis-Typen, FSME, Röteln, Hämophilus influenzae, Masern, Neisseria, Mumps, Poliomyelitis, Tetanus, Tollwut, Typhus
Sexualhormone und ihre Hemmstoffe
   Anabolika, Androgene, Antiandrogene, Gestagene, Estrogene, Antiestrogene (Tamoxifen etc.)
Zystostatika und Metastasenhemmer
   a) Alkylantien wie Nimustin, Melphalan, Carmustin, Lomustin, Cyclophosphamid, Ifosfamid, Trofosfamid, Chlorambucil, Busulfan, Treosulfan, Prednimustin, Thiotepa
   b) Antimetabolite wie Cytarabin, Fluorouracil, Methotrexat, Mercaptopurin, Tioguanin
   c) Alkaloide wie Vinblastin, Vincristin, Vindesin
   d) Antibiotika wie Aclarubicin, Bleomycin, Dactinomycin, Daunorubicin, Doxorubicin, Epirubicin, Idarubicin, Mitomycin, Plicamycin
   e) Komplexe von Nebengruppenelementen (z.B. Ti, Zr, V, Nb, Ta, Mo, W, Ru, Pt) wie Carboplatin, Cisplatin und Metallocenverbindungen wie Titanocendichlorid
   f) Amsacrin, Dacarbazin, Estramustin, Etoposid, Hydroxycarbamid, Mitoxanthron, Procarbazin, Temiposid
   g) Alkylamidophospholipide (beschrieben in J.M. Zeidler, F. Emling, W. Zimmermann und H.J. Roth, Archiv der Pharmazie, 324 (1991), 687)
   h) Etherlipide wie Hexadecylphosphocholin, Ilmofosin und Analoga, beschrieben in R. Zeisig, D. Arndt und H. Brächwitz, Pharmazie 45 (1990), 809-818.

Insbesondere sind zu nennen: Cyclosporine, wie Cyclosporin A, und Cyclosporinderivate sowie Paclitaxel.

Als Polymer kann die erfindungsgemäße Zubereitung übliche Polymere aufweisen, wie beispielsweise Polyacrylate oder Polymethacrylate (Eudragit E, L, F), Cellulosen und Cellulosederivate (Methylhydroxypropylcellulose, Ethylcellulose, Hydroxypropylcelluloseacetatsuccinat (Aquoat®) oder natürliche Polymere (Schellack, Wachse, Bienenwachs, Glanz-Wachse). Durch die Wahl des Polymers kann die Freisetzungseigenschaft der Zubereitung oder der daraus hergestellten größeren Matrixeinheiten gesteuert werden. So kann durch Einsatz von Methylhydroxypropylcellulose eine im Vergleich zu nicht retardierten Tabletten nur gering verzögerte Freisetzung des Wirkstoffs erreicht werden. Die Verwendung von Eudragit E als Polymer führt zu einer verzögerten Freisetzung des Wirkstoffs bereits im Magen. Weist die Zubereitung Eudragit L bzw. F als Polymer auf, so ist eine kontrollierte Freisetzung des Wirkstoffs erst im Darmbereich möglich.

Als Lipid kann die erfindungsgemäße Zubereitung übliche Lipide aufweisen, wie beispielsweise natürliche, halbsynthetische und synthetische Triglyceride oder deren Mischungen, Mono- und Diglyceride allein oder in Mischung untereinander oder mit z.B. Triglyceriden, natürliche und synthetische Wachse, Fettalkohole einschließlich ihrer Ester und Ether sowie Lipidpeptide. Insbesondere sind synthetische Mono-, Di- und Triglyceride als Einzelsubstanzen oder in Mischung (z.B. Hartfett), Glycerintrifettsäureester (z.B. Glycerintrilaurat, -myristat, -palmitat, -stearat und -behenat) und Wachse wie z.B. Cetylpalmitat und Cera alba (gebleichtes Wachs, DAB9), Bienenwachs (z.B. Apifil, Apifac geeignet.

Weitere Lipide, zum Teil mit zusätzlich emulgierenden (SE = self emulsifying; selbstemulgierend) Eigenschaften sind Glycerinbehenat (z.B. Compritol 888 ATO), Glycerintribehenat (Compritol 888), Palmitostearate wie z.B. Glycerinpalmitostearat (z.B. Biogapress Vegetal ATO BM 297, Precirol Ato 5, Geleol), Diethylenglykol-, Propylenglykol-, Ethylenklykol-, Polyglykol- und Propylenglykolpalmitostearat, Stearate wie Glycerinstearat (z.B. Precirol WL 2155 Ato) und Polyglykolstearat, Isostearate, Polyalkohol-Fettsäureester (z.B. Compritol WL 3284), PEG-Behenat (z.B. Compritol HD5 ATO), Cetylpalmitat (z.B. Precifac Ato), Sacharoseester wie Saccharose-Monodistearat und -Monopalmitat (z.B. Sucro-Ester W.E. 15), Saccharose-Distearat (z.B. Sucro-Ester W.E. 7), Polyglycerinester wie Polyglycerinisostearostearat (Lafil WL 3254) und -palmitostearat, Polyglykolisierte Glyceride (z.B. Gelucire, Labrafil, Suppocire), selbstemulgierendes Polyglykolstearat (z.B. Superpolystate), selbstemulgierendes Polyglykolpalmitostearat (z.B. Tefose Serie), Glyceride C₁₂-C₁₈ Fettsäuren (z.B. Lipocire) sowie deren Mischungen aus zwei oder mehr Lipiden.

Durch die Wahl des Lipids kann die Freisetzungseigenschaft der Zubereitung oder der daraus hergestellten größeren Matrixeinheiten gesteuert werden. So kann durch Einsatz von im Darm gut abbaubaren Lipiden die Freisetzung beschleunigt werden, da zusätzlich zur Freisetzung aufgrund von Diffusion aus der Matrix auch Freisetzung aufgrund vom Matrixerosion erfolgt. Mit langsamer abbaubaren Lipiden oder nicht im Magen-Darm-Trakt abbaubaren Lipiden erfolgt die Freisetzung verzögerter. Als relativ schnell durch Pankreas Lipase/Colipase abbaubares Lipid wird Dynasan 114 beschrieben, der Abbau Dynasan 118 erfolgt langsamer (C.Olbrich, R.H. Müller, Proceed. Int. Symp. Controlled Rel. Bioact. Mater., Stockholm, 921-922, 1997).

Als Hilfsstoffe können insbesondere die folgenden Stoffgruppen verwendet werden:

Füllstoffe aus dem Bereich der Zucker, wie beispielsweise Disaccharide (Laktose, Saccharose), Monosaccharide (Glukose, Fruktose) oder Polysaccharide (Stärken, Mais- oder Kartoffelstärke, Cellulose, natürliches Cellulosepulver, mikrokristalline Cellulose), Zuckeralkohole, wie beispielsweise Sorbit oder Mannit, oder Calciumphosphate.

Bindemittel, wie Polyvinylpyrrolidon (PVP, Kollidon CL), Gelatine, Stärkekleister, Cellulosen, Celluloseether oder Zucker.

Erfindungsgemäß ist festgestellt worden, daß eine Zubereitung in Form eines polymerhaltigen/lipidhaltigen Compounds, die eine Hilfsstoffphase mit wenigstens einem Hilfsstoff und/oder eine Wirkstoffphase mit wenigstens einem Wirkstoff und eine polymere phase/lipide Phase mit wenigstens einem Polymer/Lipid aufweist, wobei die Polymerphase/Lipidphase der Zubereitung inkohärent ist und die Hilfs- und/oder Wirkstoffphase kohärent ist, erhalten wird, wenn die verschiedenen Phasen der Zubereitung zusammen in einer Flüssigkeit suspendiert oder suspendiert und gelöst werden, wobei die Polymerphase/Lipidphase in der Flüssigkeit nicht löslich ist, und diese Suspension anschließend sprühgetrocknet wird.

Hierbei wird insbesondere eine Zubereitung erhalten, deren Polymerphase/Lipidphase frei von Hilfs- und/oder Wirkstoffphase ist.

Ebenso ist es möglich, die Suspension in einem Fließbett- oder Wirbelschichttrockner zu trocknen. Dabei werden die Phasen der Zubereitung wiederum zusammen in einer Flüssigkeit suspendiert oder suspendiert und gelöst, wobei die Polymerphase/Lipidphase in der Flüssigkeit nicht löslich ist, und diese Suspension wird anschließend in einem Fließbett- oder Wirbelschichttrockner getrocknet.

Zur Durchführung des erfindungsgemäßen Verfahrens werden die entsprechenden Mengen an Polymer/Lipid und Hilfsstoff und/oder Wirkstoff in einer Flüssigkeit mit Hilfe eines hochtourigen Rührers oder eines Dispergators suspendiert oder suspendiert und gelöst, wobei das Polymer/Lipid, im Gegensatz zu den bekannten Verfahren mit Polymerverarbeitung, in der Flüssigkeit nicht lösbar ist, sondern als Festoffpartikel vorliegt. In Abhängigkeit von dem zu suspendierenden Polymer/Lipid ist darauf zu achten, daß bei der Dispergierung keine zu hohen Scherkräfte und Temperaturen auftreten, die zu einer Aggregation bzw. einem Zusammenfließen von Polymerpartikeln/Lipidpartikeln führen.

Die verwendete Flüssigkeit ist insbesondere demineralisiertes Wasser oder ein wäßriges oder organisches Dispersions- bzw. Suspensionsmittel.

Die jeweils gewünschte Viskosität der im Sprühtrockner, Fließbett- oder Wirbelschichttrockner zu versprühenden Suspension wird über den prozentualen Feststoff-Anteil gesteuert. Zusätzliche Regulationsmöglichkeiten bestehen bei wasserlöslichen Hilfsstoffen über deren Konzentration und chemische Natur (z.B. Lactose, Hilfsstoffe mit ausgeprägtem viskositätserhöhenden Effekt).

Eine weitere vorteilhafte Ausgestaltung ist der Zusatz von Netzmittel und/oder Bindemittel und/oder Weichmacher (z.B. Triethylcitrat, Propylenglycol, u.a) zur Suspension. Als Bindemittel sind insbesondere Polyvinylpyrrolidon, Gelatine, Stärkekleister, Cellulose, Celluloseether oder Zucker geeignet. Sie erhöhen die mechanische Festigkeit der Zubereitung. Der Weichmacher erlaubt einen validierungsfähigen Einfluß auf die Plastizität, Verformbarkeit und Verfilmbarkeit des Polymers/Lipids und ermöglicht damit die Steuerbarkeit der Freigabe des Wirkstoffs neben dem Retard-Effekt des Polymers/Lipids an sich. Als Weichmacher können vor allem Triethylcitrat und Propylenglycol eingesetzt werden. Aber auch andere innere und äußere Weichmacher, die als übliche Zusätze zu Polymeren/Lipiden bekannt sind, sind zur Steuerung der Wirkstofffreisetzung geeignet.

Die Suspension wird anschließend bei Sprühdrücken üblicherweise über 20 bar mit Hilfe geeigneter Ein- und Mehrstoff-Düsen im Sprühturm bei geeigneten Abluft-Temperaturen, in Abhängigkeit von der Sensibilität der Wirk- und Hilfsstoffe sowie von den apparativen Gegebenheiten des Sprühturmes und dessen Peripherie, sprühgetrocknet oder im Fließbett- oder Wirbelschichttrockner getrocknet.

Die erhaltene Zubereitung kann anschließend, soweit es erforderlich ist, noch nachgetrocknet werden. Hierbei ist eine Nachtrocknung und/oder eine zusätzliche Agglomeration der Zubereitung auf Fließbett- oder Wirbelschichttrocknern möglich.

Aufgrund des Trocknungsvorgangs im Sprühtrockner, Fließbettoder Wirbelschichttrockner weist die erhaltene Zubereitung eine angenähert sphärische Form auf.

Es ist erfindungsgemäß erkannt worden, daß die beschriebene Zubereitung, die eine inkohärente Polymerphase/Lipidphase und eine kohärente Hilfsstoff- und/oder Wirkstoffphase aufweist, sich zur Verwendung bei der Herstellung von größeren Matrixeinheiten mit kontrollierten Freisetzungseigenschaften eignet. Hierbei können sämtliche bekannte Verfahren angewendet werden, so daß größere Matrixeinheiten jeder beliebigen Form erhalten werden, wie beispielsweise Tabletten, Pellets oder zylinderförmige Stäbchen. Ebenso können mit der erfindungsgemäßen Zubereitung die bekannten Verfahren zur Herstellung von Extrusionsoder Sphäronisationspellets oder zur Abfüllung der Zubereitung in Kapseln durchgeführt werden.

Ferner ist erkannt worden, daß sich die erfindungsgemäße Zubereitung insbesondere zur Herstellung von größeren Matrixeinheiten und/oder Tabletten mit kontrollierten Freisetzungseigenschaften mittels Direkttablettierung eignet. Dies ist trotz des hohen Polymeranteils/Lipidanteils der Zubereitung möglich, da durch das erfindungsgmäße Verfahren unter anderem eine sehr gute Fließeigenschaft und ein verbessertes Komprimierverhalten der Zubereitung erreicht wird.

Vorteilhaft ist insbesondere die Herstellung von Tabletten mit-. tels Direkttablettierung aus einer wirkstofffreien Zubereitung, die mit wenigstens einem Wirkstoff und bei Bedarf mit weiteren Hilfsstoffen gemischt wird, sowie aus einer wirkstoffhaltigen Zubereitung, die unter Umständen zusätzlich noch mit wenigstens einem Wirkstoff und soweit erforderlich mit weiteren Hilfsstoffen gemischt werden kann.

Neben den üblichen Tabletten sind insbesondere auch Drageekerne, Film- oder Manteltablettenkerne oder zylinderförmige Stäbchen durch Direkttablettierung bzw. direkte Komprimierung erhältlich.

Ferner kann die erfindungsgemäße Zubereitung zur Herstellung größerer Matrixeinheiten verwendet werden, die verschiedene Wirkstoffe oder den gleichen Wirkstoff in unterschiedlichen Dosen aufweisen (z.B. Schichttabletten), wobei jeder Wirkstoff oder jede Dosis einen eigenen, von den anderen Wirkstoffen oder Dosen unabhängigen Freisetzungszeitpunkt aufweist. Hierzu wird eine wirkstoffhaltige erfindungsgemäße Zubereitung, die auch zusätzlich noch wenigstens einen Hilfsstoff aufweisen kann, mit wenigstens einem weiteren oder demselben Wirkstoff, falls erforderlich unter Zusatz von Hilfsstoffen, wie beispielsweise Füll-, Formtrenn- oder Bindemitteln, gemischt. Die Mischung wird dann mittels Direkttablettierung oder nach anderen bekannten Verfahren zu größeren Matrixeinheiten verarbeitet. Dies ist besonders bei inkompatiblen Wirkstoffen vorteilhaft, da diese Vorgehensweise zu einer räumlichen Trennung der Wirkstoffe in der Arzneiform führt.

Durch die Verwendung der erfindungsgemäßen Zubereitung in einem Verfahren zur Herstellung von größeren Matrixeinheiten werden Modifikationen des Freisetzungsprofils ermöglicht, da der oder die Wirkstoffe in der größeren Matrixeinheit unterschiedlich stark als Funktion der Polymermenge/Lipidmenge eingeschlossen sind und somit unterschiedlich schnell freigesetzt werden.

Die erfindungsgemäße Verwendung der Zubereitung zur Direkttablettierung weist insbesondere den Vorteil auf, daß die Wirkstoffe und/oder Hilfsstoffe durch den angewandten Trocknungsvorgang gegenüber der herkömmlichen Feuchtgranulierung, die bisher als Vorstufe zur Komprimierung von polymerhaltigen Zubereitungen erforderlich gewesen ist, nur sehr kurze Zeit der Feuchtigkeit ausgesetzt werden. Die Temperaturbelastung ist bei den genannten Trockungsverfahren steuerbar und sogar auszuschalten, wenn im Luftstrom bei Raumtemperatur getrocknet wird.

Zur Herstellung größerer Matrixeinheiten nach bekannten Verfahren sei beispielsweise die Herstellung von Pellets angeführt. Dazu wird die erfindungsgemäße Zubereitung unter Zusatz adäquater Hilfsstoffe mit einem für die Pelletherstellung üblichen Extruder extrudiert und über eine anschließende Sphäronisation in Kügelchen von Pelletgröße überführt. Alternativ kann die Herstellung über einen Lochwalzenkompaktor mit angeschlossenem Pelltierbehälter erfolgen. Mögliche Geräte sind Spheronizer® und Marumizer®. Ebenso können diese Pellets durch Einsatz eines Pelletiertellers aus der beschriebenen Zubereitung hergestellt werden.

Diese Pellets können ebenso wie die Zubereitung selbst beispielsweise in Kapseln abgefüllt oder zu größeren Einheiten verpreßt werden.

Die Erfindung wird im folgenden anhand von Ausführungsbeispielen und Figuren näher erläutert. Alle Prozentangaben beziehen sich auf das Gewicht.

### Beispiele

### 1. Herstellung einer Lactose-Ethylcellulose-Zubereitung (50:50):

Beide Komponenten werden mit Hilfe eines Rührers in demineralisiertem Wasser dispergiert. Die Dispersion wird bei einem Feststoffgehalt von bis zu 40 Prozent und einem Sprühpumpen-Druck von 30-50 bar in einem Labor-Sprühturm bei Abluft-Temperaturen zwischen 70 und 100 Grad Celsius versprüht.

Das Ergebnis ist ein gut fließfähiges Sprüh-Agglomerat bestehend aus Lactose und Ethylcellulose in einer Korngrößenverteilung zwischen 1 und 630 µm, wobei der Hauptanteil von 50-80% zwischen 63 und 400 µm liegt.

Die so hergestellte Zubereitung zeichnet sich insbesondere aufgrund ihres Merkmals, daß die polymere Phase inkohärent und die Hilfsstoff- und/oder Wirkstoffphase kohärent ist, sowie ihrer angenähert sphärischen Form und ihrer Oberflächenbeschaffenheit (Kavitäten, Lakose) als sehr gut mit Wirkstoff misch- und beladbar aus.

Bei lipophilen Wirkstoffen kann die Freisetzungsdauer der Wirkstoffe bei direkter Mischung mit der Zubereitung bis zum Faktor drei gegenüber nicht retardierten Tabletten verlängert werden. Die Freisetzungsdauer kann wiederum durch Veränderung des Polymer-Anteiles in der Tablettiermasse, z.B. durch.Zumischen eines Füllstoffes der Typen Stärke und Laktose variiert werden.

### 2. Herstellung einer Acetylsalicylsäure (ASS) aufweisenden Lactose-Ethylcellulose-Zubereitung:

Die Herstellung erfolgt wie in 1. beschrieben, die Mischung der Komponenten Lactose:Ethylcellulose:ASS erfolgt im Gewichtsverhältnis 45:45:10.

### 3. Herstellung einer Tablette aus einer Acetylsalicylsäure (ASS) aufweisenden Zubereitung:

Die unter 1. hergestellte wirkstofffreie Lactose-Ethylcellulose-Zubereitung wird mit ASS im Verhältnis 90:10 gemischt, der Mischung wird 0,5% Aerosil und 1% Magnesiumstearat zugesetzt und direkttablettiert.

### 4. Herstellung einer Tablette aus eine Acetylsalicylsäure (ASS) aufweisenden Zubereitung:

Der unter 2. hergestellten ASS-beladenen Lactose-Ethylcellulose-Zubereitung werden 0,5% Aerosil und 1% Magnesiumstearat zugesetzt und direkttablettiert.

### 5. Herstellung einer Paracetamol-Lactose-Ethylcellulose-Zubereitung (20:40:40):

Alle Komponenten werden in demineralisiertem Wasser dispergiert und auf eine gewünschte pumpen- und druckabhängige Vikosität eingestellt. Es erfolgt die Versprühung nach den oben beschriebenen Verfahren. Die so hergestellte Zubereitung ist aufgrund ihrer Pulvereigenschaften unmittelbar für die Direkttablettierung geeignet, wobei durch den variabel einstellbaren Prozentanteil an Polymer - durch Zumischung von weiteren Hilfsstoffen, variable Tablettenhärte - die Freigabe des Wirkstoffes im gewünschten Umfang verzögert werden kann.

### 6. Herstellung eines Compritol-Trehalose-Compounds:

Compritol 888 ATO (Glyceroltribehenat) wurde geschmolzen, in heißes Wasser nach Zusatz von 1,2% Poloxamer 188 eingegossen und darin mittels eines hochtourigen Ultra-Turrax dispergiert. Nach Erkalten wurde in der wäßrigen Lipidpartikeldispersion Trehalose gelöst, so daß sich als Endkonzentration 10% Lipid und 3 % Trehalose ergab. Diese Mischung wurde in einem Mini-Büchi sprühgetrocknet (Inlet-Temperatur: 110 °C, Outlet-Temperatur: 50°C; Sprüh-Flow: 600 Normliter). Es wurde ein rieselfähiges Lipid-Hilfsstoff-Compound erhalten.

### 7. Herstellung einer Tablette aus Compound mit 1% Paracetamol:

9 Teile des in Beispiel 1 beschriebenen Lipid-Trehalose-Compounds wurde unter Zusatz von 0,1 Teil Paracetamol und unter Zumischung von 0,5% Aerosil 200 und 0,5% Magnesiumstearat auf einer Korsch-Excenterpresse direkt komprimiert. Tablettensollgewicht 505 mg.

### 8. Herstellung einer Tablette aus Compound mit 10% Paracetamol:

13 Teile des in Beispiel 6 beschriebenen Lipid-Trehalose-Compounds wurden mit 3 Teilen Trehalose gemischt, dieser Mischung 10% Paracetamol zugesetzt und unter Zumischung von 0,5% Aerosil 200 und 0,5% Magnesiumstearat auf einer Korsch-Excenterpresse direkt komprimiert. Tablettensollgewicht 505 mg.

### 9. Freisetzung aus einer Tablette aus Compound mit 10% Paracetamol:

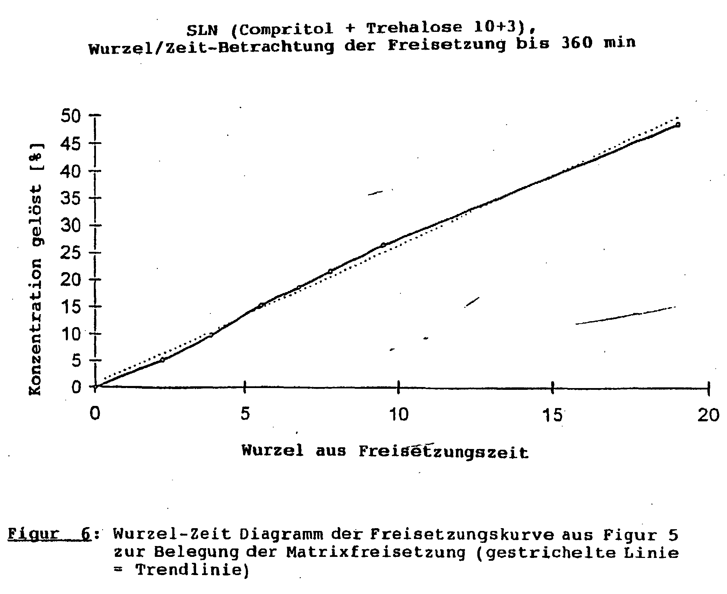

Die Freisetzung von Paracetamol aus der in Beispiel 8 hergestellten Tablette wurde mit der Paddle-Methode nach der United States Pharmacopeia bestimmt, Freisetzungsmedium: Wasser, Temperatur 37 °C. Die erhaltenen Freisetzungskurven zeigen Figur 5 und 6.

### Kurze Erläuterung der Figuren:

### Figur 1:

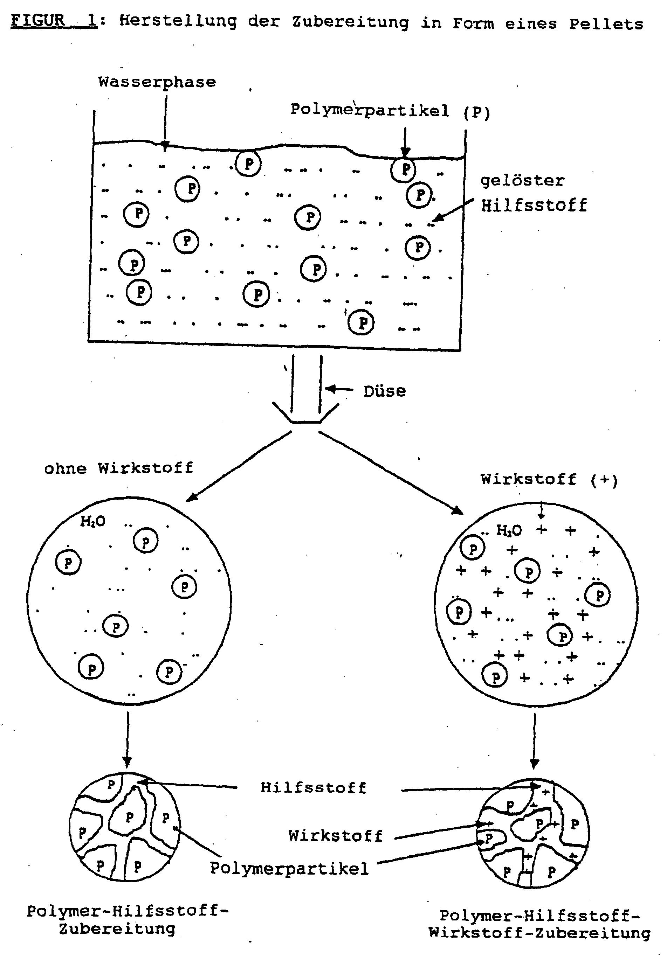

In Figur 1 ist die Herstellung einer erfindungsgemäßen Zubereitung über ein Compound nach dem erfindungsgemäßen Verfahren dargestellt: Der Matrixbildner (z.B. Polymerpartikel/Lipidpartikel) wird in Wasser dispergiert, der Hilfsstoff und/oder Wirkstoff wird ebenfalls in der Wasserphase gelöst bzw. dispergiert und die Suspension wird versprüht, wobei das Wasser durch Trocknen entfernt wird. Es entsteht eine Zubereitung, die selbst aus kleinen Polymerpartikeln/Lipidpartikeln zusammengesetzt ist, wobei die Zwischenräume mit dem Hilfsstoff (links oder mit Hilfsstoff- und Wirkstoff gefüllt sind (rechts). Die Zubereitung weist eine inkohärente polymere/lipide Phase und eine kohärente Hilfs- und/oder Wirkstoffphase auf.

### Figur 2:

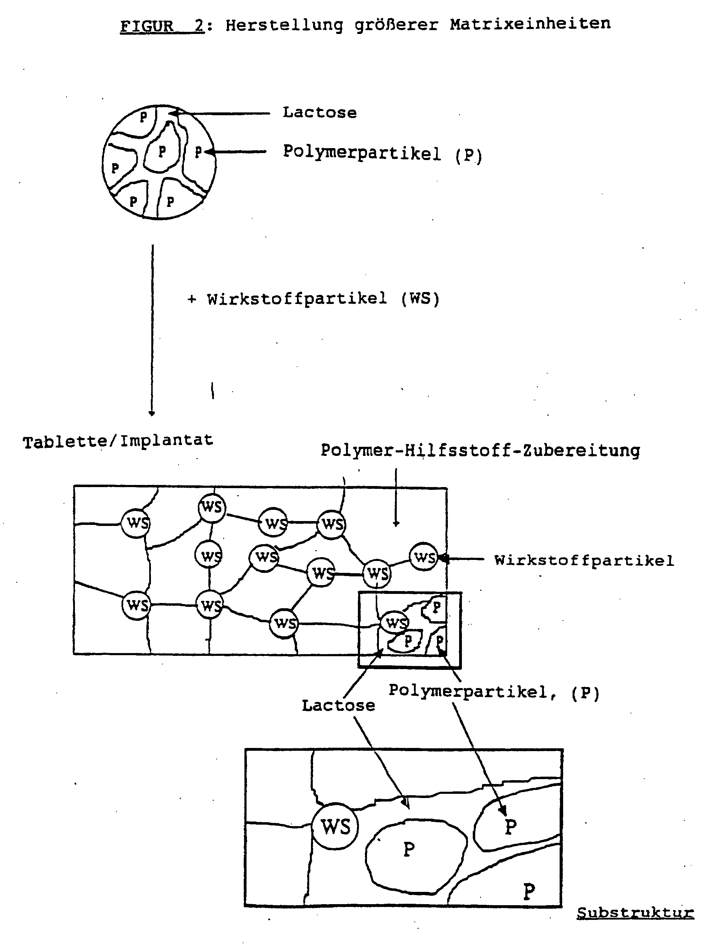

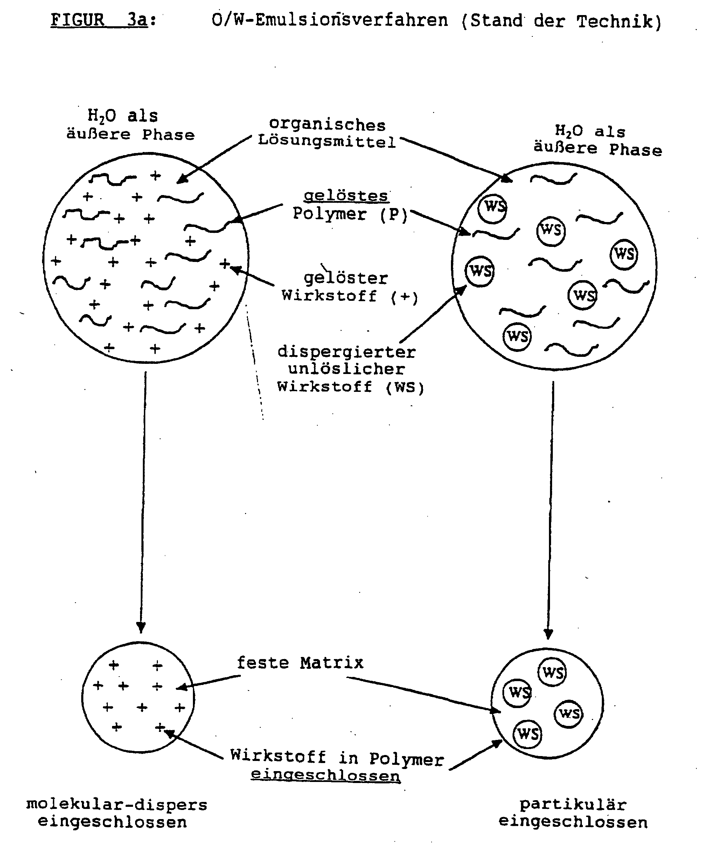

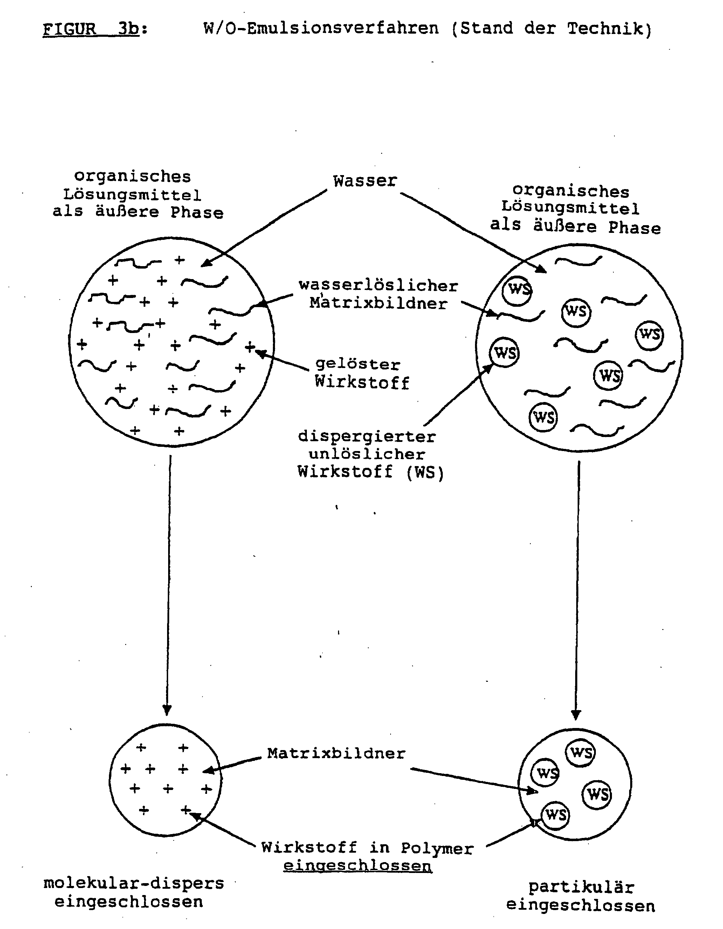

Figur 2 zeigt ein Beispiel für die Verwendung der erfindungsgemäßen Zubereitung zur Herstellung größerer Matrixeinheiten. Die wirkstofffreie Zubereitung (z.B. aus Polymer und Lactose bzw. aus Lipid und Flowlac 100- sprühgetrocknete Lactose, Fa. Meggle, Deutschland) wird mit dem Wirkstoff (in Pulverform) gemischt, gegebenenfalls Tablettierhilfsstoffe soweit erforderlich zugesetzt und die Mischung direkttablettiert.

### Figur 3a:

Aus dem Stand der Technik bekanntes O/W-Emulsionsverfahren: Hier ist ein Tropfen eines organischen Lösungsmittels mit darin gelöstem Matrixbildner (z.B. Polymer) in einer Wasserphase dispergiert (O/W-Emulsion), wobei der Wirkstoff in der organischen Phase gelöst (links) oder bei unlöslichem Wirkstoff dispergiert ist (rechts). Weitere Erklärung siehe Text.

### Figur 3b:

Aus dem Stand der Technik bekanntes W/O-Emulsionsverfahren: Hier ist ein Tropfen Wasser mit darin gelöstem Matrixbildner (z.B. wasserlösliches Polymer) in einer organischen Phase dispergiert (O/W-Emulsion), wobei der Wirkstoff in der wäßrigen Phase gelöst (links) oder bei unlöslichem Wirkstoff dispergiert ist (rechts). Weitere Erklärung siehe Text.

### Figur 4:

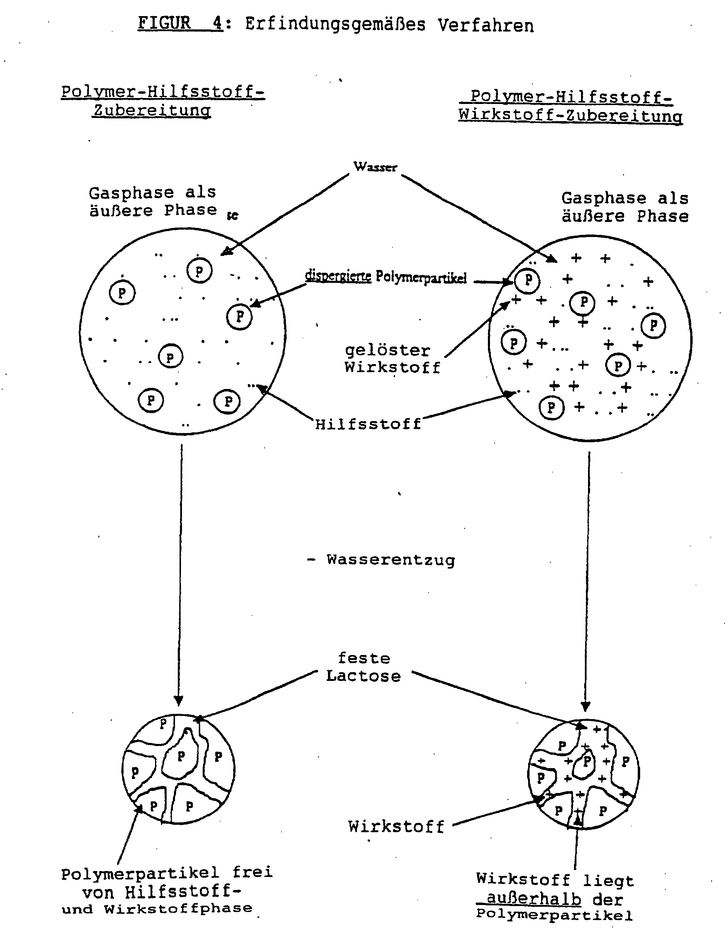

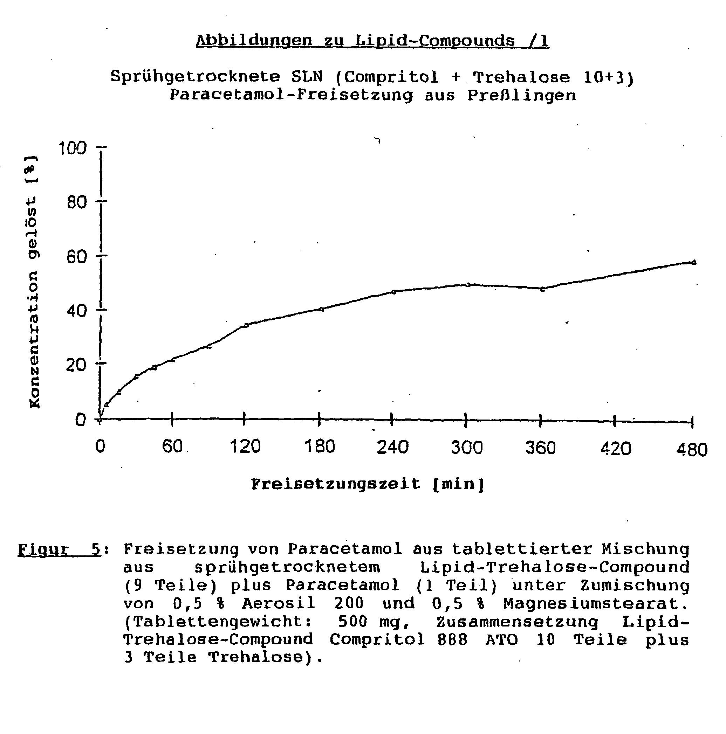

Figur 4 zeigt das erfindungsgemäße Verfahren zur Herstellung der erfindungsgemäßen Zubereitung. Die polymere Phase/Lipidphase ist nicht gelöst, sondern in Wassertropfen dispergiert bzw. suspendiert, die durch Sprühen in einer Gasphase verteilt werden. Ein Hilfsstoff (z.B. Lactose, links) oder ein Hilfsstoff und ein Wirkstoff (rechts) sind ebenfalls im Wassertropfen gelöst oder dispergiert bzw. suspendiert. Nach Entfernen des Wassers entsteht eine wirkstofffreie Hilfsstoff-Polymer/Lipid-Zubereitung (links) oder eine Wirkstoff-Hilfsstoff-polymer/Lipid-zubereitung (rechts), wobei die polymere Phase/Lipidphase in beiden Fällen inkohärent ist.

### Figuren 5 und 6:

Freisetzung von Paracetamol aus einer Tablette bei Verwendung der erfindungsgemäßen Zubereitung (Beispiel 4). Darstellung der freigesetzten Menge als Funktion der Zeit (Figur 5) und als Funktion der Wurzel aus der Zeit (Figur 6).

## Patentansprüche

1. Zubereitung in Form eines matrixmaterialhaltigen Compounds mit einer Hilfsstoffphase mit wenigstens einem Hilfsstoff und/oder einer Wirkstoffphase mit wenigstens einem Wirkstoff, **dadurch gekennzeichnet, daß** das Matrixmaterial ausgewählt ist aus Polymeren, wobei im Fall von Cellulosematerialien diese Cellulosematerialien Cellulosederivate sind, und Lipiden, die Polymerphase und/oder die Lipidphase der Zubereitung inkohärent und die Hilfs- und/oder Wirkstoffphase der Zubereitung kohärent ist.

2. Zubereitung in Form eines matrixmaterialhaitigen Compounds mit einer Hilfsstoffphase mit wenigstens einem Hilfsstoff und/oder einer Wirkstoffphase mit wenigstens einem Wirkstoff, **dadurch gekennzeichnet, daß** das Matrixmaterial ausgewählt ist aus Polymeren, wobei im Fall von Cellulose der Anteil der Matrixmaterialphase der Zubereitung 70 bis 98% beträgt, und Lipiden, die Polymerphase und/oder die Lipidphase der Zubereitung inkohärent und die Hilfs- und/oder Wirkstoffphase der Zubereitung kohärent ist.

3. Zubereitung nach Anspruch 1 oder 2, **dadurch gekennzeichnet, daß** die Matrixmaterialphase der Zubereitung Hilfs- und/oder Wirkstoff enthält oder frei davon ist.

4. Zubereitung nach einem der Ansprüche 1 bis 3, **dadurch gekennzeichnet, daß** der Anteil der Matrixmaterialphase der Zubereitung 1 bis 98% beträgt.

5. Zubereitung nach einem der Ansprüche 1 bis 4, **dadurch gekennzeichnet, daß** der Anteil der Matrixmaterialphase der Zubereitung 10 bis 95% beträgt.

6. Zubereitung nach einem der Ansprüche 1 bis 5, **dadurch gekennzeichnet, daß** der Anteil der Matrixmaterialphase der Zubereitung mehr als 15% und höchstens 90% beträgt.

7. Zubereitung nach einem der Ansprüche 1 bis 6, **dadurch gekennzeichnet, daß** der Anteil der Matrixmaterialphase der Zubereitung 40 bis 70% beträgt.

8. Zubereitung nach einem der Ansprüche 1 bis 7, **dadurch gekennzeichnet, daß** die polymere Phase ein Polyacrylat und/oder ein Polymethacrylat und/oder die Lipidphase natürliche, halbsynthetische und synthetische Triglyceride oder deren Mischungen, Mono- und Diglyceride allein oder in Mischung untereinander oder mit Triglyceriden, natürliche und synthetische Wachse, Fettalkohole einschließlich ihrer Ester und Ether sowie Lipidpeptide, insbesondere synthetische Mono-, Di- und Triglyceride als Einzelsubstanzen oder in Mischung, speziell Hartfett, Glycerintrifettsäureester, speziell Glycerintrilaurat, -myristat, -palmitat, -stearat und -behenat, und Wachse, speziell Cetylpalmitat und Cera alba (gebleichtes Wachs, DAB9), Bienenwachs enthält.

9. Zubereitung nach einem der Ansprüche 1 bis 8, **dadurch gekennzeichnet, daß** die Polymerphase ein Polyacrylat und/oder ein Polymethacrylat, ein Cellulosederivat oder natürliches Polymer und/oder die Lipidphase natürliches Lipid enthält.

10. Zubereitung nach einem der Ansprüche 1 bis 9, **dadurch gekennzeichnet, daß** sie mindestens einen Wirkstoff enthält.

11. Zubereitung nach einem der Ansprüche 1 bis 10, **dadurch gekennzeichnet, daß** die Hilfsstoffphase wenigstens einen Füllstoff, insbesondere ausgewählt aus Monosacchariden, Disacchariden, Polysacchariden, Zuckeralkoholen und Calciumphosphat, und/oder wenigstens ein Bindemittel, insbesondere ausgewählt aus Polyvinylprrolidon, Gelatine, Stärkekleister, Cellulosen, Celluloseethern und Zuckern aufweist.

12. Zusammensetzung nach einem der vorhergehenden Ansprüche, **dadurch gekennzeichnet, daß** sie in Form eines durch Direktkomprimierung herstellbaren Preßlings vorliegt.

13. Verfahren zur Herstellung einer Zubereitung in Form eines matrixmaterialhaltigen Compounds nach einem der Ansprüche 1 bis 12, **dadurch gekennzeichnet, daß** die Phasen der Zubereitung zusammen in einer Flüssigkeit suspendiert oder suspendiert und gelöst werden, wobei die Matrixmaterialphase in der Flüssigkeit nicht löslich ist, und diese Suspension anschließend sprühgetrocknet wird.

14. Verfahren zur Herstellung einer Zubereitung in Form eines matrixmaterialhaltigen Compounds nach einem der Ansprüche 1 bis 12, **dadurch gekennzeichnet, daß** die Phasen der Zubereitung zusammen in einer Flüssigkeit suspendiert oder suspendiert und gelöst werden, wobei die Matrixmaterialphase in der Flüssigkeit nicht löslich ist, und diese Suspension anschließend in einem Fließbett- oder Wirbelschichttrockner getrocknet wird.

15. Verfahren nach Anspruch 13 oder 14, **dadurch gekennzeichnet, daß** die Flüssigkeit ein wäßriges oder organisches Suspensionsmittel ist.

16. Verfahren nach einem der Ansprüche 13 bis 15, **dadurch gekennzeichnet, daß** der Suspension wenigstens ein Bindemittel und/oder wenigstens ein Netzmittel und/oder wenigstens ein Weichmacher zugesetzt wird.

17. Verwendung der Zubereitung in Form eines matrixmaterialhaltigen Compounds nach einem der Ansprüche 1 bis 12 zur Herstellung von größeren Matrixeinheiten mit kontrollierten Freisetzungseigenschaften nach bekannten Verfahren.

18. Verwendung der Zubereitung in Form eines matrixmaterialhaltigen Compounds nach einem der Ansprüche 1 bis 12 zur Herstellung von Tabletten und/oder größeren Matrixeinheiten mit kontrollierten Freisetzungseigenschaften mittels Direkttablettierung.

## Claims

1. Preparation in the form of a compound containing a matrix material with an auxiliary-substance phase comprising at least one auxiliary substance and/or an active-substance phase comprising at least one active substance, **characterized in that** the matrix material is selected from polymers, where, in the case of cellulose materials, these cellulose materials are cellulose derivatives, and lipids, where the polymer phase and/or the lipid phase of the preparation is incoherent and the auxiliary-substance phase and/or active-substance phase of the preparation is coherent.

2. Preparation in the form of a compound containing a matrix material with an auxiliary-substance phase comprising at least one auxiliary substance and/or an active-substance phase comprising at least one active substance, **characterized in that** the matrix material is selected from polymers, where, in the case of cellulose, the proportion of the matrix-material phase of the preparation is from 70 to 98%, and lipids, where the polymer phase and/or the lipid phase of the preparation is incoherent and the auxiliary-substance phase and/or active-substance phase of the preparation is coherent.

3. Preparation according to Claim 1 or 2, **characterized in that** the matrix-material phase of the preparation comprises an auxiliary substance and/or active substance or is free therefrom.

4. Preparation according to one of Claims 1 to 3, **characterized in that** the proportion of the matrix-material phase in the preparation is from 1 to 98%.

5. Preparation according to one of Claims 1 to 4, **characterized in that** the proportion of the matrix-material phase in the preparation is from 10 to 95%.

6. Preparation according to one of Claims 1 to 5, **characterized in that** the proportion of the matrix-material phase in the preparation is greater than 15% and at most 90%.

7. Preparation according to one of Claims 1 to 6, **characterized in that** the proportion of the matrix-material phase in the preparation is from 40 to 70%.

8. Preparation according to one of Claims 1 to 7, **characterized in that** the polymeric phase comprises a polyacrylate and/or a polymethacrylate, and/or the lipid phase comprises natural, semisynthetic and synthetic triglycerides or mixtures thereof, mono- and diglycerides, alone or in a mixture with one another or with triglycerides, natural and synthetic waxes, fatty alcohols, including esters thereof, and ethers, as well as lipid peptides, in particular synthetic mono-, di- and triglycerides as individual substances or in the form of a mixture, especially hydrogenated fat, glycerol trifatty acid esters, especially glycerol trilaurate, trimyristate, tripalmitate, tristearate and tribehenate, and waxes, especially cetyl palmitate and cera alba (bleached wax, DAB9), or beeswax.

9. Preparation according to one of Claims 1 to 8, **characterized in that** the polymer phase comprises a polyacrylate and/or a polymethacrylate, a cellulose derivative or natural polymer, and/or the lipid phase comprises natural lipid.

10. Preparation according to one of Claims 1 to 9, **characterized in that** it comprises at least one active substance.

11. Preparation according to one of Claims 1 to 10, **characterized in that** the auxiliary-substance phase comprises at least one filler, in particular selected from monosaccharides, disaccharides, polysaccharides, sugar alcohols and calcium phosphate, and/or at least one binder, in particular selected from polyvinylpyrrolidone, gelatine, starch glue, celluloses, cellulose ethers and sugars.

12. Composition according to one of the preceding claims, **characterized in that** it is in the form of a pressing which can be produced by direct compression.

13. Process for the production of a preparation in the form of a compound containing a matrix material according to one of Claims 1 to 12, **characterized in that** the phases of the preparation are suspended or suspended and dissolved together in a liquid, where the matrix-material phase is insoluble in the liquid, and this suspension is subsequently spray-dried.

14. Process for the production of a preparation in the form of a compound containing a matrix material according to one of Claims 1 to 12, **characterized in that** the phases of the preparation are suspended or suspended and dissolved together in a liquid, where the matrix-material phase is insoluble in the liquid, and this suspension is subsequently dried in a fluidized-bed drier.

15. Process according to Claim 13 or 14, **characterized in that** the liquid is an aqueous or organic suspension medium.

16. Process according to one of Claims 13 to 15, **characterized in that** at least one binder and/or at least one wetting agent and/or at least one plasticizer is added to the suspension.

17. Use of the preparation in the form of a compound containing a matrix material according to one of Claims 1 to 12 for the production of larger matrix units having controlled release properties by known processes.

18. Use of the preparation in the form of a compound containing a matrix material according to one of Claims 1 to 12 for the production of tablets and/or larger matrix units having controlled release properties by means of direct tabletting.

## Revendications

1. Composition sous la forme d'un composite contenant un matériau de matrice avec une phase agent auxiliaire contenant au moins un agent auxiliaire et/ou une phase principe actif contenant au moins un principe actif, **caractérisée en ce que** le matériau de matrice est choisi parmi les polymères, dans lesquels, lorsqu'il s'agit de matériaux cellulosiques, ces matériaux cellulosiques sont des dérivés de la cellulose, et parmi les lipides, et **en ce que** la phase polymère et/ou la phase lipidique de la préparation est incohérente et la phase agent auxiliaire et/ou la phase principe actif de la préparation est cohérente.

2. Composition sous la forme d'un composite contenant un matériau de matrice avec une phase agent auxiliaire contenant au moins un agent auxiliaire et/ou une phase principe actif contenant au moins un principe actif, **caractérisée en ce que** le matériau de matrice est choisi parmi les polymères, dans lesquels, dans le cas de la cellulose, la proportion de la phase matériau de matrice de la préparation est de 70 à 98%, et parmi les lipides, et **en ce que** la phase polymère et/ou la phase lipidique de la préparation est incohérente et la phase agent auxiliaire et/ou la phase principe actif de la préparation est cohérente.

3. Composition selon la revendication 1 ou 2, **caractérisée en ce que** la phase matériau de matrice de la préparation contient un agent auxiliaire et/ou un principe actif ou bien en est dépourvue.

4. Composition selon l'une des revendications 1 à 3, **caractérisée en ce que** la proportion de la phase matériau de matrice dans la préparation est de 1 à 98%.

5. Composition selon l'une des revendications 1 à 4, **caractérisée en ce que** la proportion de la phase matériau de matrice dans la préparation est de 10 à 95%.

6. Composition selon l'une des revendications 1 à 5, **caractérisée en ce que** la proportion de la phase matériau de matrice dans la préparation est supérieure à 15% et au maximum égale à 90%.

7. Composition selon l'une des revendications 1 à 6, **caractérisée en ce que** la proportion de la phase matériau de matrice dans la préparation est de 40 à 70%.

8. Composition selon l'une des revendications 1 à 7, **caractérisée en ce que** la phase polymère contient un polyacrylate et/ou un polyméthacrylate et/ou **en ce que** la phase lipidique contient des triglycérides naturels, semi-synthétiques et synthétiques ou des mélanges de ceux-ci, des mono- et diglycérides seuls ou mélangés entre eux ou avec des triglycérides, des cires naturelles et synthétiques, des alcools gras, y compris leurs esters et leurs éthers, ainsi que des peptides lipidiques, en particulier des mono-, di- et triglycérides synthétiques seuls ou en mélange, notamment des graisses durcies, des triesters d'acides gras avec le glycérol, en particulier les trilaurate, trimyristate, tripalmitate, tristéarate et tribéhénate de glycérol, et des cires, notamment le palmitate de cétyle et la cire blanche (Cera alba, DAB9), la cire d'abeilles.

9. Composition selon l'une des revendications 1 à 8, **caractérisée en ce que** la phase polymère contient un polyacrylate et/ou un polyméthacrylate, un dérivé de la cellulose ou un polymère naturel et/ou **en ce que** la phase lipidique contient un lipide naturel.

10. Composition selon l'une des revendications 1 à 9, **caractérisée en ce qu'**elle contient au moins un principe actif.

11. Composition selon l'une des revendications 1 à 10,
**caractérisée en ce que** la phase agent auxiliaire contient au moins une matière de charge, en particulier choisie parmi les monosaccharides, les disaccharides, les polysaccharides, les alcools de sucres et le phosphate de calcium, et/ou au moins un liant, en particulier choisi parmi la polyvinylpyrrolidone, la gélatine, la colle d'amidon, les celluloses, les éthers de cellulose et les sucres.

12. Composition selon l'une au moins des revendications précédentes, **caractérisée en ce qu'**elle se présente sous la forme d'une pièce moulée susceptible d'être obtenue par compression directe.

13. Procédé de fabrication d'une composition sous la forme d'un composite contenant un matériau de matrice selon l'une des revendications 1 à 12, **caractérisé en ce que** les phases de la composition sont mises en suspension ensemble dans un liquide ou bien mises en suspension et dissoutes, la phase matériau de matrice n'étant pas soluble dans le liquide, et cette suspension est ensuite séchée par pulvérisation.

14. Procédé de fabrication d'une préparation sous la forme d'un compound contenant un matériau de matrice selon l'une des revendications 1 à 12, **caractérisé en ce que** les phases de la préparation sont mises en suspension ensemble dans un liquide ou bien mises en suspension et dissoutes, la phase matériau de matrice n'étant pas soluble dans le liquide, et cette suspension est ensuite séchée dans un sécheur à lit fluidisé.

15. Procédé selon la revendication 13 ou la revendication 14, **caractérisé en ce que** le liquide est un agent de suspension aqueux ou organique.

16. Procédé selon l'une des revendications 13 à 15, **caractérisé en ce que** l'on ajoute à la suspension au moins un liant et/ou au moins un agent mouillant et/ou au moins un plastifiant.

17. Utilisation de la composition sous la forme d'un composite contenant un matériau de matrice selon l'une des revendications 1 à 12 pour la fabrication d'unités de matrice de plus grande taille ayant des propriétés de libération contrôlée selon des procédés connus.

18. Utilisation de la composition sous la forme d'un composite contenant un matériau de matrice selon l'une des revendications 1 à 12 pour la fabrication de comprimés et/ou d' unités de matrice de plus grande taille ayant des propriétés de libération contrôlée par pastillage direct.
